# EUROPEAN PATENT APPLICATION

(11) **EP 1 972 208 A1**
(43) Date of publication of application: **24.09.2008**
(21) Application number: 08004389.6
(22) Date of filing: 10.03.2008
(51) Int. Cl.: A23L 1/30, A23C 9/123, A61K 35/74, A61P 1/00

(54) **Composition for improving intestinal microflora**

(30) Priority: 16.03.2007 JP 2007068744
(71) Applicant: Kirin Holdings Kabushiki Kaisha, Tokyo 104--8288 (JP)
(72) Inventor: Nishida, Satoshic/o Koiwai Dairy Products Co Ltd Technical Development Center in Kirin Beer Yokohama Breweries, Yokohama 230-8628 (JP); Fujii, Toshioc/o Kirin Holidings Kabushiki Kaisha Central Laboratories for Frontier Technology, Yokohama 236-0004 (JP)
(74) Representative: HOFFMANN EITLE

(57) **Abstract**

The present invention is to provide a composition for improving the intestinal microflora for maintaining a good intestinal microflora. The present invention consists of a composition for improving the intestinal microflora characterized by containing *L. paracasei* KW3110 or its variant as an active component. The composition exhibits an excellent intestinal regulation effect, intestinal environment-improving effect, and intestinal microflora-improving effect, enabling to form healthy microflora in human intestine, when being ingested. Especially, a composition for improving the intestinal microflora of the present invention activates lactic acid bacteria such as *Bifidobacterium* which are beneficial bacteria among the intestinal microflora, and enables substantively the improvement of the intestinal microflora and the maintenance of a good intestinal microflora. In the present invention, it is particularly preferred that the composition is a composition wherein *L. paracasei* KW3110 or its mutant is combined with probiotics and/or prebiotics.

## Description

### Technical Field

The present invention relates to a composition for improving the intestinal microflora comprising *Lactobacillus paracasei* KW3110 or its variant as an active component, which proliferates *Bifidobacterium,* beneficial bacteria of intestinal microflora.

### Background Art

Bacteria are permanent in the human intestine, and constitute the intestinal microflora. Recently, it has been recognized that the intestinal microflora has a critical association with human health, and the interest in intestinal microflora is increasing. The constitution of the human intestinal microflora is mostly stable as long as the environment and dietary content is stable and the host is in good health. However, the ecosystem of the intestinal microflora varies depending on various factors including host age, disease, microorganism infection, stress, nutritional components, and pharmaceutical administration. Sometimes, an abnormal intestinal microflora is formed, causing detrimental effects to host's health.

Among the bacteria constituting the intestinal microflora, *Bifidobacterium* is, for example, a major genus constituting the human intestinal microflora, and it is known that *Bifidobacterium* plays various beneficial biological roles in human and animals, including growth suppression of intestinal putrefactive or pathogenic bacteria. Therefore, *Bifidobacterium* is called "beneficial bacteria" among the intestinal microflora. As *Bifidobacterium* decreases in number or disappears due to various diseases or with age etc., various attempts are made to increase intestinal *Bifidobacterium* in order to improve intestinal microflora.

As such attempts, various foods or pharmaceuticals, or *Bifidobacterium*-proliferating factors etc. are disclosed. Foods or pharmaceuticals having such object, for example, yogurt containing *Bifidobacterium* (Japanese Laid-Open Patent Application No. 2002-112703), *Bifidobacterium* powder (Japanese Laid-Open Patent Application No. 2006-280263) are disclosed. Further, as for *Bifidobacterium*-proliferating factors, those using organic solvent extraction residue of cocoa beans (Japanese Laid-Open Patent Application No. 8-196268), those using dried potatoes (Japanese Laid-Open Patent Application No. 6-217733), those using extract of Coffee Arabica plant leaf (Japanese Laid-Open Patent Application No. 6-125771), those using *Bifidobacterium* proliferative oligosaccharides (Japanese Laid-Open Patent Application No. 3-262460), those using syrup-like substances extracted from the Calonyction aculeatum fruit(Japanese Laid-Open Patent Application No. 2-135088), and those using a solvent extraction from Araliaceae (Japanese Laid-Open Patent Application No. 2-249482) are disclosed.

In order to maintain a good intestinal microflora, an improvement of intestinal microflora has been attempted by ingesting lactic acid bacteria in form of foods containing *Bifidobacterium* or probiotic agents. However, there is a problem for foods containing *Bifidobacterium,* or probiotic agents that it is difficult to maintain an effective number of viable cells in the intestine by ingesting only *Bificobacterium,* as *Bifidobacterium* is not resistant to various environmental stresses such as acid, temperature, pH, salt concentration, water activity, or bile acid. Further, as there are still problems concerning the strain to be used, viable cells count, intestinal stability, or survival and fixability in the intestinal tract, the intended effect was not always reached in the products such as foods containing *Bifidobacterium* or probiotic agents which have been conventionally developed.

Further, in order to maintain a good intestinal microflora, a method of ingesting *Bifidobacterium*-proliferating factors has been proposed. However, though a certain effect is recognized at a laboratory level for *Bifidobacterium*-proliferating factors , in many cases, a certain effect cannot be obtained when applying to humans. This is because the conditions at laboratory level and the microbiological conditions in the human intestine and of the outer world are different, and also because environmental conditions have a complex influence. On the other hand, among the *Bifidobacterium*-proliferating factors, extracts from natural products have deficits such as the complexity of the manufacturing methods, or its high costs. Moreover, *Bifidobacterium*-proliferating factors such as oligosaccharides are low molecular, and as the water content increases to enhance osmotic pressure of the intestinal content, some have side-effects such as diarrhea, which is a problem. Therefore, a method to ingest *Bifidobacterium*-proliferating factors was not satisfactory from the point of view of actual improvement of the intestinal microflora.

[Patent Document 1] Japanese Laid-Open Patent Application No. 2-135088
[Patent Document 2] Japanese Laid-Open Patent Application No. 2-249482
[Patent Document 3] Japanese Laid-Open Patent Application No. 3-262460
[Patent Document 4] Japanese Laid-Open Patent Application No. 6-125771
[Patent Document 5] Japanese Laid-Open Patent Application No. 6-217733
[Patent Document 6] Japanese Laid-Open Patent Application No. 8-196268
[Patent Document 7] Japanese Laid-Open Patent Application No. 2002-112703
[Patent Document 8] Japanese Laid-Open Patent Application No. 2006-280263

### Disclosure of the Invention

### [Object to be solved by the Invention]

The object of the present invention is to provide a composition for improving the intestinal microflora in order to maintain a good intestinal microflora, and to provide a composition for improving the intestinal microflora which enables to improve the intestinal microflora and to maintain a good microflora in a practical manner, by proliferating lactic acid bacteria such as *Bifidobacterium* which are beneficial bacteria among the intestinal microflora.

### [Means to Solve the Object]

The present inventors made a keen study to solve the above object and discovered that *L. paracasei* KW3110 or its variant proliferates lactic acid bacteria such as *Bifidobacterium* which are called beneficial bacteria among intestinal microflora. The present invention has been thus completed. By ingesting *L. paracasei* KW3110 or its variant, the human intestinal microflora in which intestinal bacteria such as *Bifidobacterium* have decreased can be improved. In other words, the present invention consists of a composition for improving the intestinal microflora comprising *L. paracasei* KW3110 or its variant as an active ingredient, wherein the composition exhibits an excellent intestinal regulation effect, intestinal environment-improving effect, and intestinal microflora-improving effect, enabling to form a healthy microflora in the human intestine, when being ingested.

The composition for improving the intestinal microflora of the present invention can proliferate *Bifidobacterium* in the intestinal microflora, and increase the *Bifidobacterium* share based on the total bacterial count of the intestinal microflora. Further, in the present invention, by making a composition for improving the intestinal microflora comprising *L. paracasei* KW3110 and its variant coexisting with probiotics and/or prebiotics as active ingredients, an especially effective improvement can be achieved in intestinal microflora, by promoting the proliferation of beneficial bacteria among the intestinal microflora while keeping the cell count of *Bifidobacterium* in intestinal microflora and activating the same.

For ingesting a composition for improving the intestinal microflora of the present invention, it can be prepared and ingested in the form of a food or drink, and yogurt can be exemplified as a preferred form of food or drink. Further, a composition for improving the intestinal microflora of the present invention can be prepared in the form of health foods including tablets, granule agent, powder agent, capsule agent, or drinkable preparations. In the present invention, it is preferred to adjust the content of lactic acid bacteria comprised in a composition for improving the intestinal microflora comprising *L. paracasei* KW3110 or its variant as an active ingredient of the present invention, to be 4 × 10⁹ or more per amount of composition for improving the intestinal microflora to be ingested per day.

That is, the present invention relates specifically to (1) use of Lactobacillus paracasei KW3110 or its variant for manufacturing a composition for a proliferation of beneficial bacteria; (2) the use of L. paracasei KW3110 or its variant according to (1), wherein the proliferation of a beneficial bacteria is a proliferation of Bifidobacterium; (3) the use of L. paracasei KW3110 or its variant according to (1), wherein the proliferation of the beneficial bacteria is an increase of Bifidobacterium share based on the total cell count of the intestinal microflora; (4) the use of L. paracasei KW3110 or its variant according to (1), comprising L. paracasei KW3110 or its variant coexisting with a probiotic and/or prebiotic as an active ingredient; (5) the use of L. paracasei KW3110 or its variant according to (4), wherein the probiotic coexisting with L. paracasei KW3110 or its variant is one or more kind of lactic acid bacteria selected from the genera Lactobacillus, Lactococcus, Streptococcus, Pediococcus and Leuconostoc.

The present invention also relates to (6) the use of L. paracasei KW3110 or its variant according to (4), wherein the prebiotic coexisting with L. paracasei KW3110 or its variant is one or more kinds of prebiotics selected from oligosaccharide, dietary fiber and yeast wall cell; (7) the use of L. paracasei KW3110 or its variant according to any one of (1) to (6), wherein the composition for a proliferation of the beneficial bacteria is prepared in the form of food or drink; (8) the use of L. paracasei KW3110 or its variant according to (7), wherein the food or drink is yogurt; (9) the use of L. paracasei KW3110 or its variant according to (7), wherein the food or drink is prepared in the form of a tablet, granule agent, powder agent, capsule agent, or drinkable preparation; (10) the use of L. paracasei KW3110 or its variant according to any one of (1) to (9), wherein the content of lactic acid bacteria in a composition for a proliferation of the beneficial bacteria comprising L. paracasei KW3110 or its variant as an active ingredient is adjusted to be 4 × 10⁹ or more per amount of composition for a proliferation of the beneficial bacteria to be ingested per day.

### [Effect of the Invention]

The present invention is to provide a composition for improving the intestinal microflora which exhibits an excellent intestinal regulation effect, intestinal environment improving effect, and intestinal microflora improving effect. By ingesting a composition for improving the intestinal microflora of the present invention, lactic acid bacteria such as *Bifidobacterium,* which are beneficial bacteria of intestinal microflora proliferate, which enables the improvement of the intestinal microflora, and to maintain a good microflora, in a practical manner. Especially, it enables to proliferate the lactic acid bacteria in the human intestine wherein a beneficial intestinal microflora such as *Bifidobacterium* has decreased or disappeared, to improve to healthy intestinal microflora.

### [Best Mode of Carrying Out the Invention]

The present invention consists of a composition for improving the intestinal microflora comprising *L. paracasei* KW3110 or its variant as an active ingredient. The composition for improving the intestinal microflora of the present invention exhibits an excellent intestinal regulation effect, intestinal environment-improving effect, and intestinal microflora-improving effect.

### (Intestinal regulation effect, intestinal environment-improving effect, and intestinal microflora-improving effect)

In the present invention, "intestinal regulation effect" is understood and defined generally to be "an effect for regulating the gastrointestinal function". An effect for regulating the gastrointestinal function relates to an improvement of defecation disturbance including constipation and diarrhea; improvement of an abdominal discomfort such as abdominal pain, abdominal distension, and feeling of unsatisfied defecation; improvement of intestinal disorders which are perceived by a subject such as abnormal movement of intestine including rolling of stomach, and excessive abdominal wind. Further, intestinal environment-improving effect means that the intestinal microflora, or a physical and chemical index such as pH or water, and metabolic products produced by the intestinal microflora are improved toward a favorable direction for the host. Intestinal microflora-improvement means that the intestinal microflora moves toward a favorable direction for the host with the proliferation of beneficial bacteria.

### (Lactic acid bacteria which are an active ingredient of the present invention)

Lactic acid bacteria having an intestinal microflora-improving effect, used as an active ingredient of the present invention, for example *L. paracasei* KW3110 can be obtained from Japan Dairy Technical Association as *L. casei* L 14 strain. Meanwhile, though there is a statement of Japan Dairy Technical Association that L14 strain is *L. casei*, when the present inventors have analyzed the strain by RFLP (Restriction Flagment Length Polymorphism) and AFLP (Amplified Flagment Length Polymorphism) using RiboPrinter (QUALICON), the strain was determined to be *L*. *paracasei,* therefore it is stated as *L. paracasei* in the present invention. *L. paracasei* KW3110 which is used as an active ingredient of a composition for improving the intestinal microflora in the present invention, can be obtained from Japan Dairy Technical Association as described in the above, and moreover, it is deposited as FERM BP-08634 at the International Patent Organism Depositary, National Institute of Advanced Industrial Science and Technology, which is an International Depositary Authority, according to Budapest Treaty on the deposit of microorganism for patent procedure.

Lactic acid bacteria, *L. paracasei* KW 3110 which is an active ingredient of the present invention, can be used as active ingredient of a food or drink of the present invention, by being cultured and proliferated appropriately in a medium for culturing lactic acid bacteria known to a skilled person such as M.R.S. (de Man, Rogosa, Sharpe) medium and the like, powdered by lyophilizing or spray-drying according to need, as viable cells. In the present invention, a mutant strain having a high intestinal microflora-improving effect can be prepared and used by mutating lactic acid bacteria having an intestinal microflora-improving effect. A means to raise mutation can be carried out by a publicly known mutating means such as UV and the like.

### (Probiotics and prebiotics used in combination)

The composition for improving the intestinal microflora of the present invention is characterized by containing *L. paracasei* KW3110 or its variant as an active ingredient, while it is preferred to be a composition wherein *L. paracasei* KW3110 or its variant is combined with probiotics and/or prebiotics. Herein, probiotics relate to "a useful microorganism that can improve the intestinal microflora and have beneficial effect to the host". Probiotics that are combined with *L. paracasei* KW3110 or its variant in the present invention, include lactic acid bacteria belonging to the genera *Lactobacillus, Lactococcus, Streptococcus, Pediococcus,* and *Leuconostoc.* Further, prebiotics relate to "a substance promoting proliferation of probiotics in the intestine". Prebiotics coexisting with *L. paracasei* KW3110 or its variant in the present invention, include oligosaccharides, dietary fibers, and yeast cell walls.

### (Intestinal microflora-improving effect of lactic acid bacteria which is an active ingredient of the present invention)

The intestinal microflora relates to a bacterial microflora which exists in the intestine. Further, improvement of the intestinal microflora means that the intestinal microflora moves toward a favorable direction for the host, as it is mentioned above. In the intestine of an individual, no less than 100 kinds, 100 billions of microbacteria exist, which are always deeply related with the host's health or diseases. Some intestinal bacteria produce a substance which causes a disease, while some of intestinal bacteria degrade and detoxify the substances. Some infect organs which they had not invaded until a moment when the resistance of the host weakens, while some activate a host's immunity to enhance the resistance. There are neutral ones which cause neither damage nor benefit. Sometimes, the constitution ratio of each bacterium in the intestinal microflora is referred to as "balance".

The balance of the intestinal microflora in an individual is generally stable and it is impossible to artificially remove a specific bacterium completely. However, it is known that the constitution ratio of bacteria in the intestinal microflora may vary when ingesting very limited substances such as probiotics, prebiotics, or antibiotics. As such, the balance of the intestinal microflora affects health condition of the host, and it is said that the "intestinal microflora has improved" when it has moved toward a favorable direction for the host.

Lactic acid bacteria which are an active ingredient of the present invention can increase the number of *Bifidobacterium* significantly contributing to the health of the host among the intestinal microflora, and the share of *Bifidobacterium* based on the total bacterial count in the intestine, by being ingested orally by a human as viable cells. *Bifidobacterium* is one of predominant bacteria among the human intestinal microflora. The lactic acid bacteria of the present invention may change the potency of predominant bacteria of the intestinal microflora, and change the balance of the intestinal microflora in a favorable direction for the host.

### (Use in fermented food or drink)

A composition for improving the intestinal microflora of the present invention can be prepared in a form of food or drink. For example, by adding *L. paracasei* KW3110 or its variant which is an active ingredient to raw materials of food or drink, and allowing fermentation, a fermented food or drink can be prepared and used. When using a composition for improving the intestinal microflora of the present invention as a fermented food or drink, it should just have an effective dose of the active ingredients when it is made as a fermented product.

Here, the term "effective dose of the active ingredients" relates to the content wherein the active ingredients are ingested within the following range, when the amount generally consumed for each food or drink is ingested.
In other words, the intake of the effective dose of the active ingredients of the present invention in food or drink can be determined depending on the recipient, the age and body weight of the recipient, symptoms, administered time, form of food or drink, ingesting methods, etc.

When the intake of the effective dose of the active ingredients of the present invention in food or drink is indicated by the number of the lactic acid bacteria, it is preferred that the intake is 4 × 10⁹ or more per day, more preferably 1 × 10¹⁰ or more per day, most preferably 4 × 10¹⁰ or more per day. Therefore, the number of the lactic acid bacteria to be contained per each food is determined according to the amount of food or drink generally ingested per day. For example, when ingesting yogurt as a fermented food, the active ingredients of the present invention can be compounded to the food so that the intake will be within 50 - 500 g per day for an adult, and preferably within 60 - 200 g.

The field of fermented foods using the lactic acid bacteria is roughly classified into dairy products, meats, breads, beverages and vegetables, based on examples of its utilization, heretofore. When manufacturing food or drink using the lactic acid bacteria, it is possible to add an intestinal microflora-improving effect to the food or drink by using the lactic acid bacteria having an intestinal microflora-improving effect of the present invention as lactic acid bacteria or a part thereof.

### (Use by compounding into food or drink)

As another embodiment when preparing a composition for improving the intestinal microflora of the present invention in the form of a food or drink, the lactic acid bacterium which is an active ingredient can be added to the food or drink and used without fermentation of lactic acid bacteria. To use a composition for improving the intestinal microflora of the present invention by compounding into food or drink, the intake of the effective dose of the active ingredients to the food or drink is added and compounded during the stage of raw material or after the product is manufactured and the like. Here, the term "effective dose of the active ingredients" relates to the intake necessary to exert an effect of the present invention, and the effective dose is as described in the above. Further, it is preferred to pulverize lactic acid bacteria for example, by means of freeze dry (FD) or spray dry (SD). Furthermore, it is preferred that food or drink having an intestinal microflora-improving effect of the present invention is manufactured in the form of a food or drink filled in a sealed container, in order to prevent contamination of other microorganisms, and to keep the quality of the content.

As another embodiment when preparing a composition for improving the intestinal microflora of the present invention in the form of a food or drink, it can be prepared as health foods, functional foods, specified health foods, or patient foods, by adding a composition for improving the intestinal microflora of the present invention to add an intestinal microflora-improving effect. Moreover, it is not limited to a form of food, and it can be in a form of beverage having an intestinal microflora-improving effect. As active ingredients of the present invention have an intestinal microflora-improving effect, it is possible to provide foods that can be ingested continuously and have an intestinal microflora-improving effect, by compounding an active ingredient of the present invention to foods ingested daily, or to health foods or functional foods and the like ingested as supplements. Further, the lactic acid bacteria with an intestinal microflora-improving effect of the present invention can be used by compounding in food or drink such as tea, drinkable preparations, or tablets. Further, according to the present invention, by replacing whole or a part of the lactic acid bacteria of beverages or foods manufactured using lactic acid bacteria or that of beverages of foods comprising lactic acid bacteria, with lactic acid bacteria having an intestinal microflora-improving effect of the present invention, it is possible to make beverages or foods with an intestinal microflora-improving effect.

When compounding the lactic acid bacteria with an intestinal microflora-improving effect of the present invention to beverages, the amount of lactic acid bacteria to be compounded can be determined appropriately. Generally an amount that can be ingested continuously as beverage, and is able to exert sufficiently an intestinal microflora-improving effect of the lactic acid bacteria is applied as an amount to be compounded. When the intake of the effective dose of the active ingredients of the present invention to food or drink is indicated by the number of the lactic acid bacteria, it is as mentioned in the above. For example, when 100 g of beverage is ingested per day, it is preferred to add 4 × 10⁹ or more of bacteria per 100 g of beverage. On the other hand, when considering not to damage the flavor or appearance of the beverage by adding lactic acid bacteria, an amount of 4 × 10¹¹ or less is preferred. Moreover, an amount of 10¹¹ or less is more preferred. Therefore, the concentration of lactic acid bacteria having an intestinal microflora-improving effect, and being stable, having good taste and good storage ability is most preferred to be within 10⁹ - 10¹¹ bacteria per 100 g of beverage. Meanwhile, as for the relationship between the number of the lactic acid bacteria and the weight of dried bacteria, for example, for *L. paracasei* KW3110 , 10¹² bacteria correspond to 1 g of dry weight.

In the present invention, as for a beverage compounded with the lactic acid bacteria having an intestinal microflora-improving effect of the present invention, various beverages can be exemplified, and for manufacturing these, any of saccharide, flavor, juice, food additives and the like used for general beverage formulation can be used. As for manufacturing beverages, existing reference books, for example "Revised new edition: soft drinks" (Kohrin) and the like, can be referred to.

Moreover, as form of the product, a form of beverage in a sealed container used generally as product form of beverages is particularly preferred, and as for sealed container, any form of can, bottle, PET bottle, paper container can be used. Moreover, there is no particular limitation for the volume, and it can be determined generally by considering the amount that a consumer ingests daily as beverage, the number of the lactic acid bacteria to be compounded, and the bacterial count which is necessary per day.

### (Use in formulation form of a food or drink)

As an embodiment when preparing a composition for improving the intestinal microflora of the present invention in the form of a food or drink, it is possible to prepare it in formulation form of a food or drink. Various formulation forms of food or drink can be exemplified, and usually used formulation forms can be exemplified as formulation forms of food or drink as health foods and functional foods. When manufacturing a food or drink in the formulation form, adjuvants such as an excipient, extender, binding agent, disintegrant, lubricant, dispersant, preservative, wetting agent, solving adjuvant, antiseptic, stabilizer, capsule base material and the like can be used besides food materials and food additives generally used. Examples of the adjuvants include: lactose, fructose, glucose, starch, gelatin, magnesium carbonate, synthetic magnesium silicate, talc, magnesium stearate, calcium carbonate, methylcellulose, carboxymethylcellulose, or salt thereof, gum arabic, polyethylene glycol, syrup, vaserine, glycerin, ethanol, propylene glycol, citric acid, sodium chloride, sodium sulfite, sodium phosphate, pullulan, carrageenan, dextrin, reduced palatinose, sorbitol, xylitol, stevia, artificial sweetener, citric acid, ascorbic acid, acidulant, sodium bicarbonate, sucrose ester, vegetable hydrogeneted oil, potassium chloride, safflower oil, bees wax, soybean lecithin, flavor and the like. As for the manufacture of such health foods and functional foods, reference books on drug formulation, for example "Practical guide of Japanese Pharmacopoeia (General rule on formulation)" (Hirokawa Shoten) and the like can be referred to.

In the present invention, as a form of a food or drink in a formulation suitable for health foods and functional foods, a form of a tablet, capsule, granule, powder, suspension, or emulsion can be exemplified. Examples of a method for manufacturing health foods and functional foods in a form of a tablet include a manufacturing method by compressing a product compounding lactic acid bacteria having an intestinal microflora-improving effect of the present invention to a certain form, or a method wherein the mixture is wetted with a solvent such as water or alcohol and is formed in a certain form or poured into a certain mold. Examples of methods for manufacturing health foods and functional foods in the form of a capsule include a method of filling a product compounding lactic acid bacteria having an intestinal microflora-improving effect of the present invention into capsules in the form of a liquid, suspension, paste, powder or granule, or a manufacturing method by encapsulating and forming with capsule base materials, such as hard capsules or soft capsules and the like.

The present invention will be explained in detail in the following, but the present invention will not be limited by these.

### [Example 1]

### (Test design)

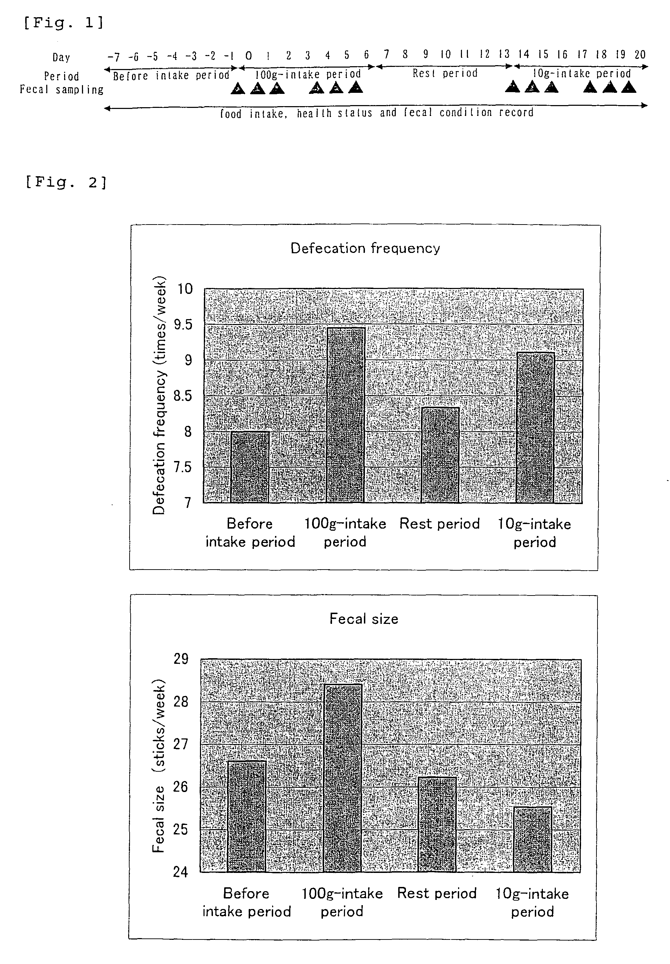

The following human test was performed according to the Helsinki declaration, with an informed consent. The test subjects were 9 healthy Japanese men and women, having a regular defecation, and generally defecating daily. Male-female ratio of test subjects was 2 men and 7 women. Background of the test subjects was as follows: age 27.1 ± 6.8 (mean ± standard deviation), BMI 21.6 ± 1.9 (mean ± standard deviation). Test subjects had an interview with a doctor at the initiation and termination of the test. The schedule of the present test is shown in Fig. 1.

Test period was 28 days, Day -7 - Day -1 was set as "before intake period", a period during which a yogurt containing 4 × 10⁸ cfu/g or more of *L. paracasei* KW3110 as viable cells (hereinafter referred to as KW yogurt) is not ingested; Day 0 - Day 6 was set as "100 g-intake period", a period during which 100 g of KW yogurt is ingested per day; Day 7 - Day 13 was set as "rest period" , a period during which no KW yogurt is ingested; Day 14 - Day 20 was set as "10 g-intake period", a period during which 10 g of KW yogurt is ingested per day. During the test period, intake of foods affecting intestinal microflora other than KW yogurt, such as yogurt, lactic acid bacteria beverage, oligosaccharide, dietary fiber, fermented soybeans (natto), and pharmaceuticals affecting intestinal tract was limited. Fecal samples were collected from every test subjects once a day, every day during Day 1 - Day 5 and Day 13 - Day 19, except Day 2 and Day 16, and day without defecation. The composition in 100 g of yogurt is shown in Table 1.

**[Table 1]**

| (per 100g) | |
|---|---|
| Energy | 90.1kcal |
| Protein | 3.5g |
| Fat | 3.1g |
| Carbohydrate | 12.2g |
| Sodium | 51.0mg |
| *Lactobacillus paracasei* strain KW3110 | 4×10¹⁰ cfu |

### (Treatment of fecal sample)

Feces were collected and kept being refrigerated under anaerobic conditions by using Aneropack Kenki, and were subjected to a microflora analysis by the cultivation method within 24 hours. Further, feces were stored at -20°C and subjected to microflora analysis by a molecular biology method such as the realtime PCR method.

In the present example, culturing methods were performed according to a method of Mitsuoka et al. Specifically, feces were homogenized, suspended to a saline solution supplemented with 0.09% agar, diluted gradually and applied to a plane medium, to measure each bacterial count. Bacteria to be a target of detection include total anaerobes, total aerobes, *Bifidobacterium* and *Lactobacillus.* EG agar medium and BL agar medium were used for total anaerobes, TS agar medium for total aerobes, BL agar medium and TOS propionate agar medium for *Bifidobacterium,* and LBS agar medium for *Lactobacillus.* Total bacterial count was set to be the sum of total anaerobes count and total aerobes count. Share of *Bifidobacterium* was set to be the ratio of *Bifidobacterium* based on the total bacterial count in the cultivation method.

### (DNA extraction)

In the present example, DNA extraction was conducted as follows: 2 ml of suspended fecal sample was washed three times with Extraction buffer (100 mM Tris, 50 mM EDTA, pH 9.0), the supernatant was recovered to make a pellet. Further, a standard sample for realtime PCR was harvested to extract DNA, after counting cells count of *Bifidobacterium* longum JCM1217 strain cultured in GAM medium (Nissui), *Lactobacillus* gaseri JCM1131 cultured in M.R.S. medium (Oxoid), and *L. paracasei* 3110 strain with a microscope. DNA extraction was performed with Fast DNASPIN Kit for soil (Qbiogene), according to an instruction manual.

### (Realtime PCR)

Realtime PCRs were all performed by using Light Cycler (Roche). 5 µL of template DNA solution was added to 15 µL of Master Mix (Takara, SYBR^{®} Premix Ex Taq^{™} (Perfect Real Time) 1x Master Mix, each primer 0.2 µM, 0.1 µg/µL BSA), to prepare 20 µL of PCR reaction solution. References of primers used for detecting each of intestinal bacterium in a sample and specific DNA sequences are as follows.
Further, for writing a standard curve necessary for quantification, a serially diluted standard sample DNA solution for realtime PCR, which was mentioned above, was used.

Determination of *Bifidobacterium* was performed by referring to Applied and Environmental Microbiology, 68:5445-5451, by using g-Bifid-F : 5-CTC CTG GAA ACG GGT G-3; g-Bifid-R; 5-GGT GTT CTT CCC GAT ATC TAC A-3. Determination of *Lactobacillus* was performed by referring to Journal of Microbiological Methods, 51:313-321, by using LactoF: 5-TGGAAACAGRTGCTAATACCG-3; LactoR: 5-CCATTGTGGAAGATTCCC-3. Determination of *L. paracasei* was performed by referring to Letters in Applied Microbiology, 29:90-92, by using
PARA:5-CACCGAGATTCAACATGG-3;
Y2:5-CCCACTGCTGCCTCCCGTAGGAGT-3.

PCR reaction conditions were as follows: after a pre-incubation at 94°C for 10 sec, 40 cycles were performed under dissociation/annealing/elongation conditions according to each primer, and after the elongation reaction, SYBR Green fluorescence was measured. Temperature conditions of each cycle was: for determination of *Bifidobacterium,* 15 sec at 95°C, 10 sec at 55°C, 25 sec at 72°C; for determination of *Lactobacillus,* 15 sec at 95°C, 20 sec at 60°C, 20 sec at 72°C; for determination of *L. paracasei,* 15 sec at 95°C, 10 sec at 45°C, 25 sec at 72°C. Specificity of PCR products was confirmed by performing dissociation curve analysis. Dissociation curve analysis conditions were as follows: after PCR amplification reaction, the reaction solution was incubated for 0 sec at 95°C, after keeping the temperature of 50°C for 10 sec, SYBR Green fluorescence was measured continuously while increasing the temperature slowly up to 95°C at a rate of 0.2°C/sec. Quantitative analysis and dissociation curve analysis were both performed with Light Cycler Software version 5.32. Observational days were Day-1, Day 0, Day 1, Day 3, Day 5, Day 13, and Day 18. Six test subjects whom fecal sample deletion was small at these observational days were made as a subject. Bacterial count of intestinal microflora was set to be the number in 1 g of wet weight of feces.

### (Detection of Lactobacillus paracasei)

*Lactobacillus parcasei* was detected from feces by combining cultivation method and colony PCR method (RAPD-PCR method). In other words, genes were recovered by colony morphology, from a *Lactobacillus* colony grown in LBS agar medium of cultivation method, and detected by RAPD-PCR method with a *L. paracasei* specific primer. Primer sequence was estimated to be PARA:S-CACCGAGATTCAACATGG-3; Y2:
5-CCCACTGCTGCCTCCCGTAGGAGT-3. Amplified products were electrophoresed in a 1.0% agarose gel, and analyzed.

### (Questionnaire and statistical analysis)

Test subjects recorded every day in a questionnaire form fecal conditions including defecation frequency and fecal size, intake conditions of limited foods or pharmaceuticals, dietary life or physical conditions. Fecal size was compared with that of a model of 2 cmØ × 5 cm. Data were added for each period. Comparison of total bacterial count, total anaerobes, total aerobes, *Bifidobacterium, Lactobacillus, L. paracasei,* defecation frequency, defecation output, was performed by using corresponding Wilcoxon signed rank sum test. Detection ratios of *L. paracasei* from feces were compared with χ² test. P < 0.05 was determined to be a significant difference. SPSS 11.0 J was used as statistical software.

### (Test results)

Detection results of *L. paracasei* according to the cultivation method and the colony PCR method (herein after referred to as cultivation methods), and detection results of *L. paracasei* by realtime PCR method are shown in Table 2.

**[Table 2]**

| Detection of *L. paracasei* from feces | | | | | | | |
|---|---|---|---|---|---|---|---|
| | | Before intake period | 100g-intake period | | | | |
| | | Day-1 | Day0 | Day1 | Day3 | Day4 | Day5 |
| Cutivation method | Cell count (cfu/g) | 1.65 ± 0.12 ^{a} | 09 ± 0.24 | 6,97 ± 0,13 | 7,36 ± 0,36 | 6,98 ± 0,06 | 6,96 ± 0,91 |
| | Detection ratio | 1/9^{b}(11%)^{c} | 6/8 (75%)* | 4/7 (57%)* | 8/8 (100%)* | 7/8 (88%)* | 7/7 (100%)* |
| Real-time PCR method | Cell count (cells/g) | N.D. | 8,79 ± 0,80 | 9,07 ± 0,84 | 9,34 ± 0,16 | - | 9,06 ± 0,00 |
| | Detection ratio | 0/6 (0%) | 6/6 (100%) | 6/6 (100%) | 5/5 (100%) | - | 6/6 (100%) |
| | | Rest period | 10g-intake period | | | | |
| | | Day13 | Day14 | Day15 | Day17 | Day18 | Day19 |
| Cutivation method | Cell count(cfu/g) | 2,12 ± 0,60 | 4,66 ± 0,12 | 6,74 ± 0,10 | 6,96 ± 0,33 | 6,79 ± 0,25 | 5,40 ± 0,44 |
| | Detection ratio | 1/9 (11%) | 2/9 (22%) | 4/7 (57%)** | 4/7 (57%)** | 6/9 (67%) ** | 4/6 (67%)** |
| Real-time PCR method | Cell count(cfu/g) | 4,77 ± 0,94 | - | - | - | 8,39 ± 0,31 | - |
| | Detection ratio | 3/6 (50%) | - | - | - | 6/6 (100%) | - |

| | | | | | | | |
|---|---|---|---|---|---|---|---|
| ^{a} Log10(Mean±S.D.) ^{b} Number of *L. paracasei* detection/Number of fecal samples. ^{c} Detection ratio of *L. paracasei.* * Significantly different compared with Day-1. (*p*<0.05) ** Significantly different compared with Day13. (*p*<0.05) | | | | | | | |

According to the cultivation method, *L*. *paracasei* was not detected in 8 test subjects out of 9 on Day-1, and was detected in an amount of 10^{6.92-7.42} cfu/g during the 100 g-intake period. In the remaining 1 test subject, *L. paracasei* was detected in an amount of 10^{2.60} cfu/g at Day-1, and 10^{7.08} cfu/g at Day 1. In all 9 test subjects, *L. paracasei* was detected in an amount of 10^{1.65} cfu/g at Day 1, and 10^{6.96-7.36} cfu/g throughout the 100 g-intake period. *L. paracasei* was not detected in 8 test subjects out of 9 at Day 13, and an amount of 10^{4.70-7.01} cfu/g was detected throughout the 10 g-intake period. In the same test subject in which *L. paracasei* was detected at Day-1, an amount of 10^{3.08} cfu/g was detected at Day 13, and 10^{4.01-6.25} Cfu/g throughout the 10 g-intake period. In all 9 test subjects, *L*. *paracasei* was detected in an amount of 10^{2.12} cfu/g at Day 13, and in an amount of 10^{4.66-6.96} cfu/g throughout the 10 g-intake period.

According to the realtime PCR method, in any of 6 test subjects including the 1 test subject in which *L. paracasei* was detected at Day-1 with the cultivation method, no *L. paracasei* was detected at Day-1 of before intake period. In all of the 6 test subjects, an amount of 10^{8.79-9.34} cells/g of L. paracasei was detected throughout the 100 g-intake period. In 3 test subjects, no *L*. *paracasei* was detected at Day 13, and 10^{8.17} cells/g of *L*. *paracasei* was detected at Day 18. In the other 3 test subjects, 10^{5.07} cells/g of *L. paracasei* was detected at Day 13, and 10^{8.17} cells/g of *L*. *paracasei* was detected at Day 18. In all of the 6 test subjects, 10^{4.77} cells/g of *L. paracasei* was detected at Day 13, and 10^{8.39} cells/g of *L*. *paracasei* was detected at Day 18.

As *L. paracasei* was detected in a high cell count drastically after the 100 g-intake period during which KW yogurt containing a high amount of KW3110 strain was ingested, it was considered that *L. paracasei* which was detected during the 100 g-intake period, 10 g-intake period, and during the rest period with the realtime PCR method, was *L*. *paracasei* KW3110. From this consideration, *L. paracasei* which was detected during the 100 g-intake period, 10 g-intake period, and during the rest period with the realtime PCR method, will be referred to as *L. paracasei* KW3110.

Meanwhile, there is a difference of approximately 10² cells/g for the detected cell count of *L. paracasei* KW3110 measured by the cultivation method and by the realtime PCR method, and the detection rate is also different. Mainly 3 possibilities can be estimated for its reason. The first one, the selected pressure of the selected medium in the cultivation method; the second one, the difference of detection sensitivity for predominant and recessive bacteria in the cultivation method; and the third one is the detection of dead bacteria in the realtime PCR method. The results of variation per day of intestinal microflora are shown in Table 3.

**[Table 3]**

| Change in intestinal flora | | | | | | |
|---|---|---|---|---|---|---|
| | Before intake period | 100g-intake period | | | | |
| (Cultivation method) | Day-1 | Day0 | Day1 | Day3 | Day4 | Day5 |
| Total | 10,54 ± 0,25^{a} | 10,58 ± 0,30 | 10,61 0,30 | 10,72 ± 0,36 | 10,54 ± 0,13 | 10,59 ± 0,44 |
| Total Anaerobes | 10,53 ± 0,25 | 10,58 ± 0,29 | 10,61 0,30 | 10,72 ± 0,36 | 10,53 ± 0,12 | 10,59 ± 0,44 |
| Total Aerobes | 8,45 ± 0,78 | 8,39 ± 0,51 | 8,35 0,49 | 8,46 ± 0,80 | 8,56 ± 0,96 | 8,06 ± 0,33 |
| *Bifidobacterium* | 9,90 ± 0,70 | 9,94 ± 0,86 | 10,06 0,91 | 10,12 ± 0,85* | 9,97 ± 0,74 | 10,05 ± 0,80* |
| *Lactobacillus* | 5,68 ± 0,81 | 7,22 ± 0,30* | 6,99 0,12 | 7,36 ± 0,36* | 6,98 ± 0,06* | 6,99 ± 0,93* |
| (Real-time PCR method) | | | | | | |
| *Bifidobacterium* | 10,24 ± 0,13 | 10,47 ± 0,50 | 10,39 ± 0,31 | 10,50 ± 0,24 | | 10,51 ± 0,54 |
| *Lactobacillus* | 8,65 ± 1,00 | 8,47 ± 0,20 | 8,70 ± 0,46 | 8,94 ± 0,79 | | 8,89 ± 0,80 |
| (Cultivation method) | Rest period | 10g-intake period | | | | |
| | Day13 | Day14 | Day15 | Day17 | Day18 | Day19 |
| Total | 10,58 ± 0,24 | 10,71 ± 0,50 | 10,70 ± 0,32 | 10,71 ± 0,51 | 10,53 ± 0,29 | 10,56 ± 0,30 |
| Total Anaerobes | 10,58 ± 0,23 | 10,67 ± 0,52 | 10,69 ± 0,32 | 10,70 ± 0,51 | 10,54 ± 0,26 | 10,55 ± 0,29 |
| Total Aerobes | 8,55 ± 0,50 | 8,44 ± 0,58 | 8,33 ± 0,54 | 8,64 ± 0,94 | 8,62 ± 0,83 | 8,66 ± 0,92 |
| *Bifidobacterium* | 9,90 ± 0,64 | 9,96 ± 0,77 | 10,15 ± 0,93 | 10,10 ± 0,94 | 9,95 ± 0,68 | 9,94 ± 0,36 |
| *Lactobacillus* | 5,63 ± 0,85 | 6,27 ± 0,52 | 6,92 ± 0,23 | 7,14 ± 0,37** | 7,09 ± 0,34** | 5,70 ± 0,67 |
| (Real-time PCR method) | | | | | | |
| *Bifidobacterium* | 10.45 ± 0.45 | - | - | - | 10.60 ± 0.56 | - |
| *Lactobacillus* | 8.19 ± 0.26 | - | - | - | 8.30 ± 0.35 | - |

| | | | | | | |
|---|---|---|---|---|---|---|
| ^{a} Log10(Mean±S.D.) * Significantly different compared with Day-1. ( *p*<0.05) ** Significantly different compared with Day13. ( *p*<0.05) | | | | | | |

In the cultivation method, *Lactobacillus* increased significantly at Day 0, Day 3, Day 4 and Day 5 compared to Day-1 during the 100 g-intake period. Similarly, *Bifidobacterium* increased at Day 3, and Day 5 compared to Day-1. During the 10 g-intake period, *Lactobacillus* increased significantly at Day 17 and Day 18 compared to Day 13, and the mean level of *Bifidobacterium* increased in the same manner as in the 100 g-intake period.
In the realtime PCR method, *Lactobacillus* showed an increasing tendency while having no significant difference during Day 0 - Day 5 compared to Day-1. This was similar for Day 13 and Day 18. For *Bifidobacterium,* there was no significant difference at Day 0, Day 1, Day 3, Day 5 compared to Day-1, while the mean level increased. This was similar for Day 13 and Day 18.

The questionnaire results are shown in Fig. 2. During the 100-g intake period, the mean levels of both defecation frequency (Fig. 2-a) and quantity of feces (Fig. 2-b) were higher compared to those before the intake period, rest period, and 10 g-intake period, while having no significant difference.

### [Example 2]

### (Test design)

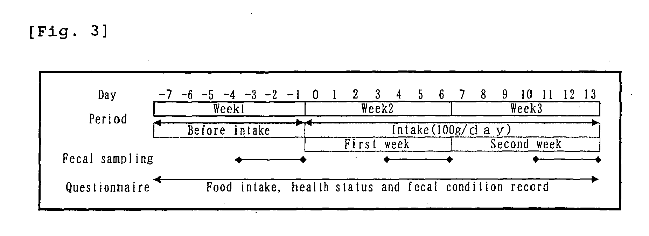

The test subjects were 10 healthy Japanese female adults, which tended to be constipated. The background of the test subjects was as follows: age 22.90 ± 3.57 (mean ±standard deviation), BMI 20.55±2.75 (mean ± standard deviation). The test subjects had an interview with a doctor at the initiation and termination of the test. The schedule of the present test is shown in Fig. 3. Test period was 21 days, and Day-7 - Day-1 was set as "before intake period" during which no KW yogurt is ingested. Day 0 - Day 13 was set as "intake period" during which KW yogurt is ingested in an amount of 100 g per day. Among this period, Day 0 - Day 6 was set as "first week", and Day 7 - Day 13 as "second week". During the test period, intake of foods affecting the intestinal microflora including yogurt other than KW yogurt, lactic acid bacteria beverage, oligosaccharide, dietary fiber, fermented soybeans (natto), and pharmaceuticals affecting the intestinal tract was limited. Fecal samples were collected for 3 times, once between Day-3 - Day-1 of the before the intake period, once between Day 4 - Day 6 of the intake period first week, and once between Day 11 - Day 13 of the intake period second week.

### (Treatment of fecal samples)

Feces were collected and kept being refrigerated under anaerobic conditions by using Aneropack Kenki, and were subjected to a microflora analysis by the cultivation method within 24 hours. Further, feces were stored at -20°C, and subjected to a microflora analysis by a molecular biology method such as the realtime PCR method. The cultivation method in the present example was performed according to a method of Mitsuoka et al. similarly as Example 1. Bacteria to be a target of detection include total anaerobes, total aerobes, *Bifidobacterium* and *Lactobacillus.* EG agar medium and BL agar medium were used for total anaerobes, TS agar medium for total aerobes, BL agar medium and TOS propionate agar medium for *Bifidobacterium,* and LBS medium for *Lactobacillus.* Total bacterial count was set to be the sum of total anaerobes count and total aerobes count.

In the present example, realtime PCR was performed with the same conditions as in the realtime PCR of Example 1. All of the collected fecal samples were set as target. Bacterial count of intestinal microflora was set to be the number in 1 g of wet weight of feces.

### (Detection of Lactobacillus paracasei)

Detection of *Lactobacillus paracasei* from feces was performed by combining the cultivation method and the colony PCR method (RAPD-PCR method) similarly as in Example 1. Amplified products were electrophoresed in 1% agarose gel and analyzed.

### (Questionnaire and statistical analysis)

Test subjects recorded every day in a questionnaire form fecal conditions including defecation frequency, fecal size, defecation sensation, fecal color, fecal shape; intake conditions of limited foods or pharmaceuticals; dietary life or physical conditions. Fecal size was compared with that of a model of 2 cmØ × 5 cm. Defecation sensation was scored in 4 levels. Fecal color and fecal shape were compared with a sample sheet. Fecal color was scored in 6 scales according to JIS Colors Standard-Glossy Edition, 5Y8/12 (yellow), 2.5Y7/12 (pale ochre), 10YR5/8 (ochre), 7.5YR4/6 (brown), 5Y4/4 (dark brown), and 2.5GY4/3 (dark brown verge on black). Fecal shape was scored as follows in 6 levels: very hard; hard; banana-like; unformed stool; muddy stool; watery stool. For defecation frequency and fecal size, the total sum of the before intake period and each week of intake periods were calculated. For defecation sensation, fecal color, and fecal shape, the scores were averaged respectively for the before intake period, and each week of intake periods.

Comparison of total bacterial count, total anaerobes, total aerobes, *Bifidobacterium, Lactobacillus, Clostridium, L. paracasei,* defecation frequency, fecal size, defecation sensation, fecal color and fecal shape for each period, was performed by using a corresponding Wilcoxon signed rank sum test. Detection ratios of *L*. *paracasei* from feces were compared with χ² test. P < 0.05 was determined to be a significant difference. SPSS 11.0 J was used as statistical software.

### (Test results)

The detection results of *L. paracasei* by the cultivation method and the colony PCR method (herein after referred to as cultivation methods), and the detection results of *L. paracasei* by realtime PCR methods are shown in Table 4.

**[Table 4]**

| Detection of *L. paracasei* (strain KW3110) from feces | | | | |
|---|---|---|---|---|
| | | Before intake period | Intake period first week | Intake period second week |
| Cutivation method | Cell count (cfu/g) Detection ratio | n.d. ^{a} 0/10^{c} (0%)^{d} | 7,96 ± 0,40 ^{b} * 5/10 (50%) * | 7,92 ± 0,40 * 10/10 (100%) * |
| Realtime-PCR method | Cell count(cells/g) Detection ratio | 5,57 ± 0,25 3/10 (30%) | 9,39 ± 0,26 * 10/10 (100%) * | 9,36 ± 9,36 * 10/10 (100%) * |

| | | | | |
|---|---|---|---|---|
| ^{a} Did not detected ^{b} Log10(Mean±S.D.) ^{c} Number of *L. paracasei* strain KW3110 detection/Number of fecal samples. ^{d} Detection rate of *L. paracasei* strain KW3110. * Significantly different compared with before intake period. (*p*<0.05) | | | | |

According to the cultivation method, *L. paracasei* was not detected during the before intake period, and was detected in an amount of 10^{7.96} cfu/g during the intake period first week, and 10^{7.92} cfu/g during the intake period second week. There was a significant difference between the intake period first week and intake period second week compared to the before intake period. According to the realtime PCR method, *L. paracasei* cell count was detected in an amount of 10^{5.57} cells/g during the before intake period, 10^{9.39} cells/g during the intake period first week, and 10^{9.36} cells/g during the intake period second week. There was a significant difference between the intake period first week and intake period second week compared to the before intake period. Meanwhile, *L*. *paracasei* was detected in 3 test subjects during the before intake period. The average of L. paracaseicell count of these 3 test subjects was 10^{5.57} cells/g during the before intake period, 10^{9.48} cells/g during the intake period first week, and 10^{9.48} cells/g during the intake period second week. The average of L. paracasei cell count of the remaining 7 test subjects was 10^{9.34} cells/g during the intake period first week, and 10^{9.30} cells/g during the intake period second week. As an average of all of the test subjects, the *L. paracasei* cell count during the intake period first week and intake period second week, was 6000 times or more than that of the before intake period. Thus, a significantly high number of *L*. *paracasei* was detected during the intake period first week and intake period second week compared to the before intake period, according to both cultivation method and realtime PCR method. Therefore, it was estimated that *L. paracasei* KW3110 contained in KW yogurt in a large amount which was ingested orally was detected.

Therefore, *L*. *paracasei* which was detected during the intake period first week and intake period second week, will be referred to as *L*. *paracasei* KW3110. According to the cultivation method, the detection ratio of *L. paracasei* KW3110 was 50% during the intake period first week, and 100% during the intake period second week. On the other hand, it was considered to be 0% during the before intake period. There was a significant difference during the intake period first week and intake period second week, compared to the before intake period. According to the realtime PCR method, the detection ratio of *L. paracasei* KW3110 was 100% during the intake period first week, and 100% during the intake period second week. The results of variation per day of intestinal microflora are shown in Table 5.

**[Table 5]**

| | Before intake | Intake period first | Intake period second |
|---|---|---|---|
| (Cultivation method) | period | week | week |
| Total | 10,46 ± 0,40^{a} | 10,42 ± 0,24 | 10,71 ± 0,41 *** |
| Total Anaerobes | 10,45 ± 0,40 | 10,36 ± 0,23 | 10,63 ± 0,35 ** |
| Total Aerobes | 8,32 ± 0,54 | 8,58 ± 0,94 | 8,65 ± 0,10 |
| *Bifidobacterium* | 9,63 ± 0,60 | 9,88 ± 0,77 | 10,28 ± 0,31 * ** |
| *Lactobacillus* | 6,45 ± 0,91 | 7,96 ± 0,40 * | 7,93 ± 0,40 ** |
| *Clostridium* | 8,85 ± 1,00 | 8,42 ± 0,52 * | 8,23 ± 0,22 ** |
| | | | |
| (Realtime-PCR method) | | | |
| *Bifidobacterium* | 10,43 ± 0,38 | 10,57 ± 0,45 * | 10,68 ± 0,59 * |
| *Lactobacillus* | 7,22 ± 0,37 | 8,99 ± 0,80 * | 8,91 ± 0,66 * |

| | | | |
|---|---|---|---|
| ^{a} Log10(Mean±S.D.) * Significantly different compared with before intake period. (*p*<0.05) ** Significantly different compared with intake period first week. (*p*<0.05) | | | |

According to the cultivation method, *Lactobacillus* increased significantly during the intake period first week and intake period second week, compared to the before intake period. The mean levels of the intake period first week and the intake period second week were approximately similar, having no significant differences. Meanwhile, *Bifidobacterium* increased significantly during the intake period second week compared to the before intake period, and during the intake period second week compared to the intake period first week. Compared to the before intake period, there was an increasing tendency during the intake period first week (p = 0.066). The total bacterial count increased significantly during the intake period second week compared to the before intake period, and during the intake period second week compared to the intake period first week. Total anaerobes count increased significantly during the intake period second week compared to the intake period first week.

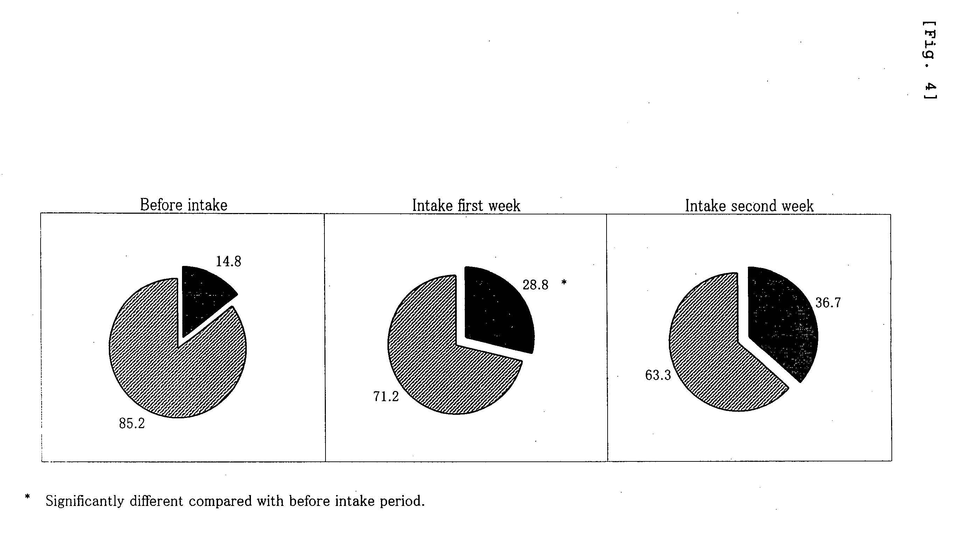

According to the realtime PCR method, *Lactobacillus* increased significantly during the intake period first week and intake period second week, compared to the before intake period. The averages of the intake period first week and the intake period second week were approximately similar, having no significant differences. *Bifidobacterium* increased significantly during the intake period first week and intake period second week compared to the before intake period. There was no significant difference between the intake period first week and intake period second week. As it is shown in Fig. 4, the share of *Bifidobacterium* increased significantly during the intake period second week compared to the before intake period. Further, there was an increasing tendency during the intake period first week compared to the before intake period (p = 0.053).

Next, the questionnaire results are shown in Table 6. The defecation frequency had an increasing tendency while there was no significant difference among all of the test subjects. There were 5 test subjects whose defecation frequency was 4 times or less per week during the before intake period, and for these test subjects the average of defecation frequency increased. The fecal size had an increasing tendency while there was no significant difference. The fecal size per defecation increased significantly during the intake period first week compared to the before intake period. The defecation sensation improved significantly during the intake period first week compared to the before intake period.

**[Table 6]**

| | | Before intake period | Intake period first week | Intake period Second week |
|---|---|---|---|---|
| Defecation frequency | (All subjects n=10) | 4,5 ± 1,4^{a} | 4,6 ± 2,0 | 4,7 ± 2,3 |
| Defecation frequency | (Mild constipated subjects^{†} n=5) | 3,4 ± 0,9 | 4,4 ± 1,8 | 5,0 ± 2,9 |
| Fecal size | | 11,8 ± 4,0 | 14,0 ± 4,0 | 12,4 ± 3,9 |
| Fecal size/one defecation | | 2,8 ± 1,1 | 3,5 ± 1,5 * | 2,9 ± 0,9 |
| Refreshing feeling | | 2,6 ± 0,5 | 2,2 ± 0,7 * | 2,4 ± 0,6 |
| Fecal color | | 4,2 ± 0,7 | 3,9 ± 0,7 | 4,0 ± 0,8 |
| Fecal shape | | 3,0 ± 1,1 | 2,7 ± 0,8 | 3,0 ± 1,1 |

| | | | | |
|---|---|---|---|---|
| ^{a} Log10 Mean±S.D. ^{†} Subjects whose defecation frequency was less than four times in before intake period. * Significantly different compared with before intake period.(p<0.05) | | | | |

### [Brief Description of Drawings]

[Fig. 1] It is a figure showing the schedule of the intake test of yogurt containing *L*. *paracasei* KW3110, in the Example of the present invention.
[Fig. 2] It is a figure showing the questionnaire results of the intake test of yogurt containing *L*. *paracasei* KW3110, in the Examples of the present invention. 2-a is a figure showing the results of defecation frequency (times/week) in the yogurt-intake test, and 2-b is a figure showing the results of defecation output (number of sample sticks/week) in the yogurt-intake test.
[Fig. 3] It is a figure showing the schedule of intake test of yogurt containing *L. paracasei* KW3110, in the Example of the present invention.
[Fig. 4] It is a figure showing the share of Bifidobacterum, with the variation per day of intestinal microflora during the intake test of yogurt containing L. *paracasei* KW3110 in the Example of the present invention.

## Claims

1. Use of Lactobacillus paracasei KW3110 or its variant for manufacturing a composition for a proliferation of beneficial bacteria.

2. The use of L. paracasei KW3110 or its variant according to claim 1, wherein the proliferation of a beneficial bacteria is a proliferation of Bifidobacterium.

3. The use of L. paracasei KW3110 or its variant according to claim 1, wherein the proliferation of the beneficial bacteria is an increase of Bifidobacterium share based on the total cell count of the intestinal microflora.

4. The use of L. paracasei KW3110 or its variant according to claim 1, comprising L. paracasei KW3110 or its variant coexisting with a probiotic and/or prebiotic as an active ingredient.

5. The use of L. paracasei KW3110 or its variant according to claim 4, wherein the probiotic coexisting with L. paracasei KW3110 or its variant is one or more kind of lactic acid bacteria selected from the genera Lactobacillus, Lactococcus, Streptococcus, Pediococcus and Leuconostoc.

6. The use of L. paracasei KW3110 or its variant according to claim 4, wherein the prebiotic coexisting with L. paracasei KW3110 or its variant is one or more kinds of prebiotics selected from oligosaccharide, dietary fiber and yeast wall cell.

7. The use of L. paracasei KW3110 or its variant according to any one of claims 1 to 6, wherein the composition for a proliferation of the beneficial bacteria is prepared in the form of food or drink.

8. The use of L. paracasei KW3110 or its variant according to claim 7, wherein the food or drink is yogurt.

9. The use of L. paracasei KW3110 or its variant according to claim 7, wherein the food or drink is prepared in the form of a tablet, granule agent, powder agent, capsule agent, or drinkable preparation.

10. The use of L. paracasei KW3110 or its variant according to any one of claims 1 to 9, wherein the content of lactic acid bacteria in a composition for a proliferation of the beneficial bacteria comprising L. paracasei KW3110 or its variant as an active ingredient is adjusted to be 4 × 10⁹ or more per amount of composition for a proliferation of the beneficial bacteria to be ingested per day.


SEQUENCE LISTING

<110> KIRIN HOLDINGS KABUSHIKI KAISHA

<120> COMPOSITION FOR IMPROVING INTESTINAL MICROFLORA

<130> 2007-0005

<160> 6

<170> PatentIn version 3.1

<210> 1
<211> 17
<212> DNA
<213> Artificial

<220>
<223> PCR primer for quantification of Bifidobacterium

<400> 1
ctcctggaaa cgggtgg 17

<210> 2
<211> 22
<212> DNA
<213> Artificial

<220>
<223> PCR primer for quantification of Bifidobacterium

<400> 2
ggtgttcttc ccgatatcta ca 22

<210> 3
<211> 21
<212> DNA
<213> Artificial

<220>
<223> PCR primer for quantification of Lactobacillus

<400> 3
tggaaacagr tgctaatacc g 21

<210> 4
<211> 18
<212> DNA
<213> Artificial

<220>
<223> PCR primer for quantification of Lactobacillus

<400> 4

ccattgtgga agattccc                                                    18

/

<210>  5
<211>  18
<212>  DNA
<213>  Artificial

<220>
<223>  PCR primer for quantification of L. paracasei

<400>  5
caccgagatt caacatgg                                                    18


<210>  6
<211>  24
<212>  DNA
<213>  Artificial

<220>
<223>  PCR primer for quantification of L. paracasei

<400>  6
cccactgctg cctcccgtag gagt                                             24